# EUROPEAN PATENT APPLICATION

(11) **EP 3 614 148 A1**
(43) Date of publication of application: **26.02.2020**
(21) Application number: 18190190.1
(22) Date of filing: 22.08.2018
(51) Int. Cl.: G01N 33/564

(54) **NOVEL BIOMARKERS**

(71) Applicant: Bianchi, Marco Emilio, 20133 Milano (IT); Manfredi, Angelo Andrea, 20124 Milano (IT); Maugeri, Norma Iris, 20125 Milano (IT)
(72) Inventor: The designation of the inventor has not yet been filed
(74) Representative: Teuten, Andrew John

(57) **Abstract**

The present invention provides *inter alia* the use of a platelet-derived microparticle expressing HMGB1 as a biomarker for the diagnosis of systemic sclerosis, assessment of severity and progression of systemic sclerosis and assessment of the efficacy of therapy for systemic sclerosis.

## Description

### FIELD OF THE INVENTION

The invention relates to biomarkers and methods of diagnosing and treating systemic sclerosis.

### BACKGROUND OF THE INVENTION

Systemic sclerosis (also known as systemic scleroderma and diffuse scleroderma, SSc) is an autoimmune disease with a chronic and progressive course. Tissue fibrosis interferes with the architecture and with the function of organs and tissues. The mechanisms initiating and sustaining the disease are poorly understood. As a consequence, there are no effective therapies, and there is a complete lack of biomarkers that can inform clinicians on the progression of the disease and help predict the rate of clinical deterioration (Denton et al., 2017). All clinical decisions are currently taken on the basis on functional performance of the organs affected by SSc. Thus the availability of biomarkers to assess the severity, the aggressivity and the progression of SSc remains an unmet medical need.

Dysfunctional and dysregulated response to tissue injury contributes to the pathogenesis of the disease. Activated fibroblasts and myofibroblasts are effectors of fibrosis and their heterogeneity of properties, regulatory pathways and origin contributes to clinical diversity among patients (Gilbane et al., 2013).

Activation of endothelial cells, loss of capillaries and exposure of sub-endothelial connective tissues are early events in systemic sclerosis. At later stages vessels undergo remodelling with thickening of the medial layer, hyperplasia of the intima and eventual obliteration of the vascular lumen. This is accompanied by abnormal expression of transcription factors, cytokines and growth factors and by defects of angiogenesis and of vasculogenesis. The microvascular damage results in tissue hypoxia and oxidative stress and contributes to myofibroblast activation and fibrogenesis. The mechanisms underpinning endothelial injury are elusive (Gabrielli et al., 2009).

Concomitant to the changes in the systemic sclerosis endothelial lining, platelets undergo activation and release growth factors like VEGF and PDGF, transforming growth factor-β (TGF-β) and serotonin. Platelets are also a source of the High Mobility Group Box-1 (HMGB1) protein (Rouhiainen et al., 2000), a Damage Associated Molecular Pattern (DAMP) (Harris et al., 2012). HMGB1 promotes fibrogenesis after damage of endothelial cells and favours tissue remodelling (Hayakawa et al., 2012).

Platelet HMGB1 activates neutrophils and commits them to generate neutrophil extracellular traps (NETs). NETs are a source of autoantigens and a player in the pathogenesis of autoimmune diseases Systemic Lupus Erythematosus and Rheumatoid Arthritis (Grayson et al., 2016). However, little is known on neutrophil potential involvement in systemic sclerosis, save that neutrophilia in bronchoalveolar lavage and neutrophilic infiltration within lung alveoli and *interstitium* correlate with the presence and the extent of lung fibrosis and disease severity (Silver et al., 1984), possibly representing the only hint to a direct involvement of neutrophils in tissue damage.

### SUMMARY OF THE INVENTION

The inventors have found that patients with systemic sclerosis have in peripheral blood high levels of platelet-derived microparticles that expose HMGB1 on their surface, autophagic and proteolytic neutrophils and byproducts from neutrophil extracellular trap (NET). These HMGB1⁺ platelet-derived microparticles activate neutrophils *in vitro* and *in vivo,* committing them to a state characterized by redistribution of granule content, autophagy-dependent prolonged survival and generation of NETs. When transferred in immunocompromised mice, HMGB1⁺ platelet-derived microparticles from the blood of SSc patients cause endothelial damage and lung fibrosis, indicating that they play a role in SSc causation and progression. Consequently, these microparticles have been found to be useful as a biomarker for systemic sclerosis.

Thus, according to a first aspect of the invention, there is provided the use of a platelet-derived microparticle expressing HMGB1 as a biomarker for the diagnosis of systemic sclerosis, assessment of severity and progression of systemic sclerosis and assessment of the efficacy of therapy for systemic sclerosis.

Aspects of the invention include methods for the diagnosis of systemic sclerosis, assessment of severity and progression of systemic sclerosis and assessment of the efficacy of therapy for systemic sclerosis and products useful therein.

The invention also provides other related methods, uses, kits and antibodies.

### BRIEF DESCRIPTION OF THE FIGURES

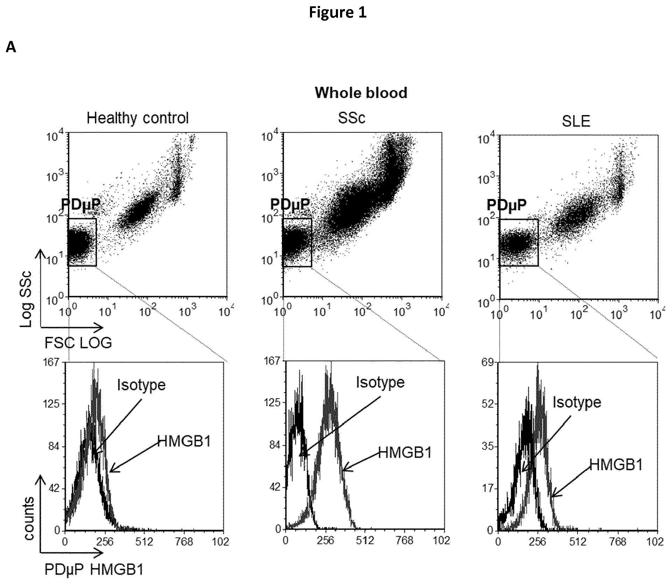

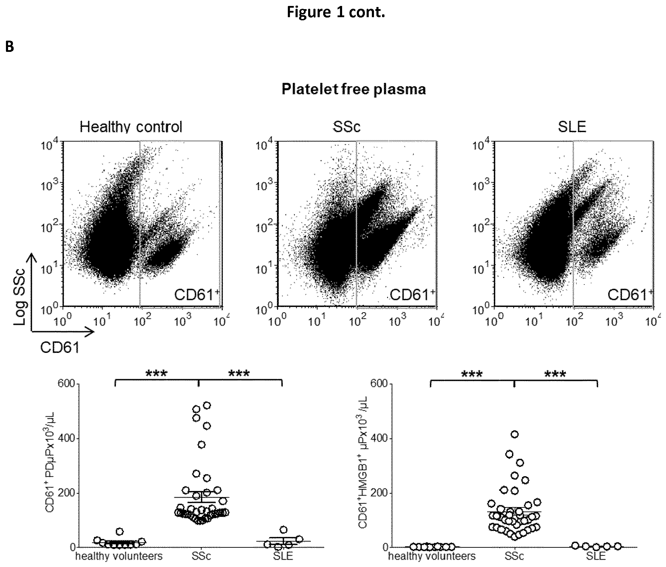

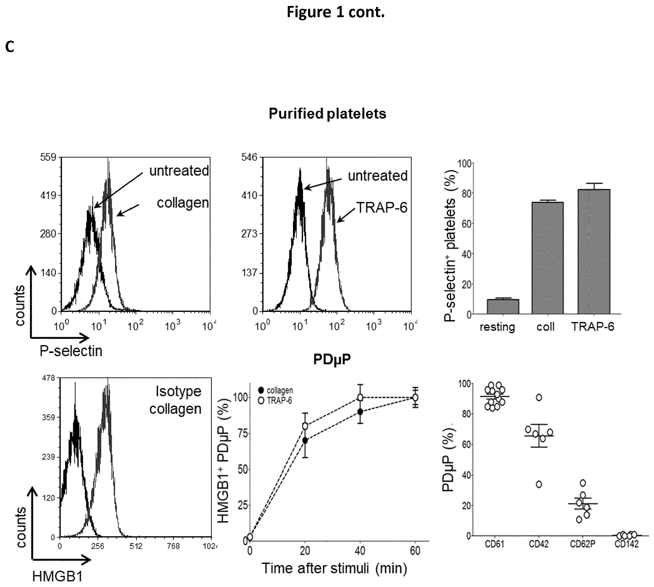

Figure 1: Platelet microparticles from patients with SSc express HMGB1
   A: Platelet-derived microparticles in whole blood from patients with active systemic sclerosis (SSc), healthy subjects, and patients with SLE were stained with antibodies against HMGB1 and with isotype-matched irrelevant antibodies.
   B: The upper panels show the platelet-derived microparticles that were identified in platelet-free plasma based on side scatter (SSc) and CD61 expression. The lower panels show the concentration of platelet-derived CD61⁺ microparticles (left) and of CD61⁺ microparticles expressing HMGB1 (right). Each dot corresponds to a single subject and lines indicate means ± SEM. ***, p<0.0001.
   C: Representative profiles showing platelet P-selectin expression after stimulation with collagen or TRAP-6. Bars indicate means±SEM (five different experiments carried out independently). Lower panels: left, expression of HMGB1 on microparticles released by platelets stimulated with collagen for 5 minutes; central panel: fraction of HMGB1⁺ microparticles released by collagen- or TRAP-6-stimulated platelets at various times; right: expression of CD61, CD42, CD62P (P-selectin) and CD142 (Tissue Factor) on collagen-microparticles. Each dot represents the result of a single independent assessment and lines indicate mean ± SEM.

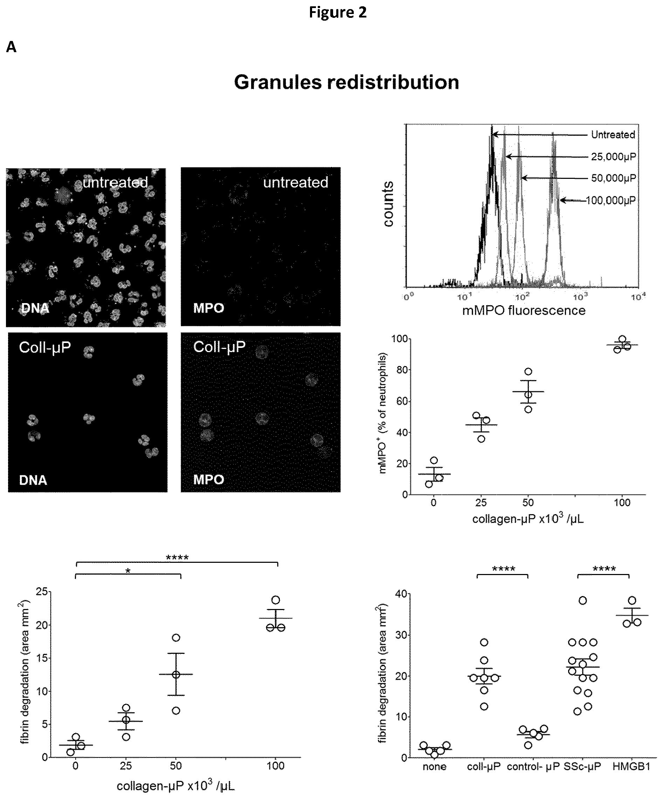

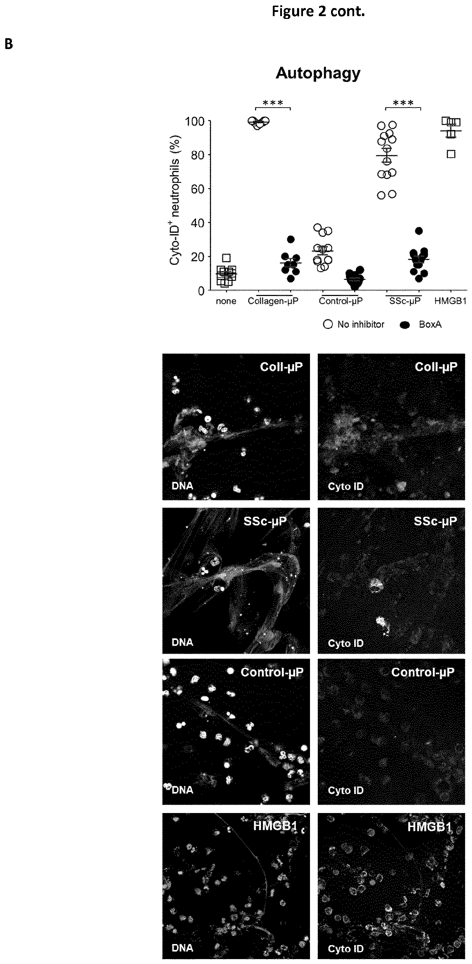

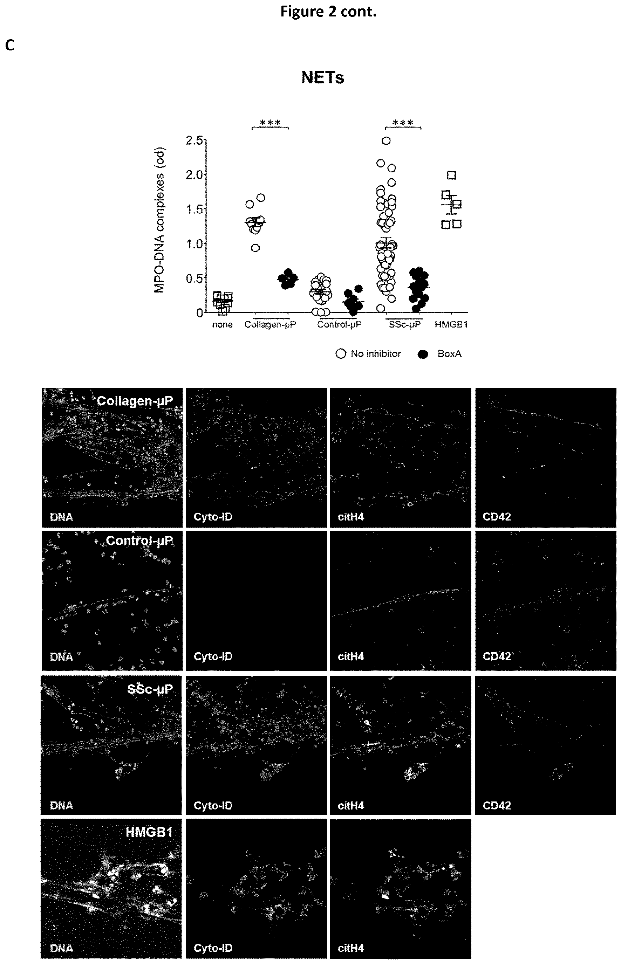

Figure 2: Microparticles activate neutrophils via HMGB1.
   A: neutrophils from healthy donors were stimulated or not with collagen-microparticles and membrane MPO expression (mMPO) was assessed by confocal microscopy and flow cytometry. ,Shown is a representative experiment of ten carried out independently. Upper histogram: MPO fluorescence intensity; middle graph: fraction of neutrophils expressing mMPO after stimulation of neutrophils with increasing amounts of collagen-microparticles. Lower graphs: fibrin proteolysis. Lower left graph: actual area of fibrin proteolysis (mm²) by neutrophils stimulated with increasing amounts of collagen-microparticles. Lower right graph: effect of 100,000 microparticles/µL of different origin (collagen-, or derived from SSc patients or healthy controls) on the ability of neutrophils to digest fibrin. Recombinant HMGB1 was used as positive control. Each dot represents a single donor. Lines indicate mean±SEM. *, p<0.05; ****, p<0.0001 by ANOVA and Bonferroni's test.
   B. Top: neutrophils were stimulated with collagen-, control- or SSc-microparticles, or with HMGB1, and analyzed by confocal microscopy for the Cyto-ID autophagy tracer. Cyto-ID was quantified by flow cytometry in untreated ("none") or microparticle-stimulated neutrophils with (black circles) or without (open circles) the HMGB1 inhibitor, BoxA. HMGB1 is the positive control. ***, p<0.0001 by ANOVA and Bonferroni's test.
      Bottom: Autophagosomes were visualized by confocal microscopy.
   C. The concentration of the NET byproducts, MPO-DNA complexes, released by neutrophils was assessed by ELISA (top) and NETs were visualized by confocal microscopy (bottom). ****, p<0.0001 by ANOVA and Bonferroni's test.

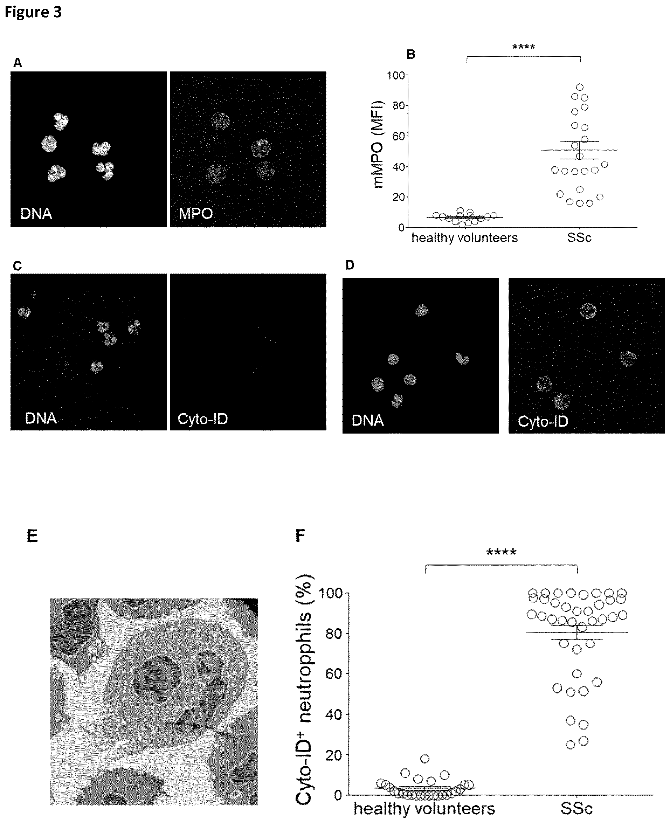

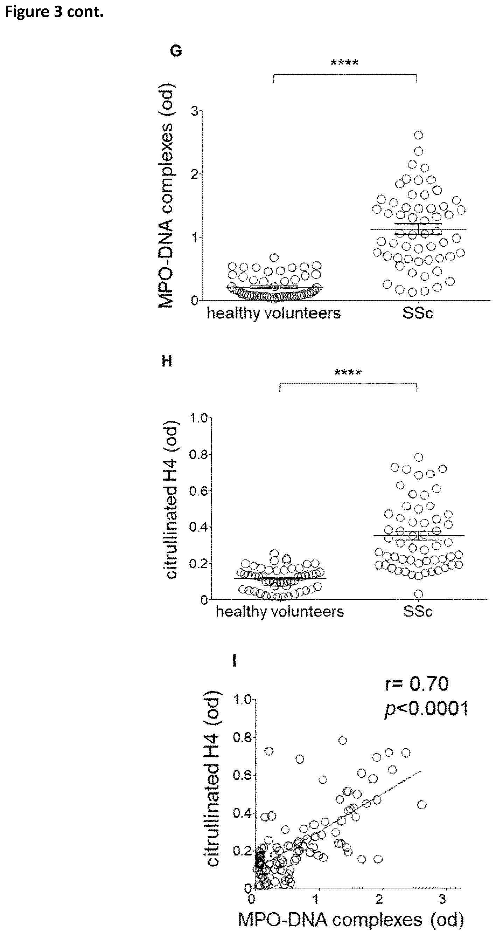

Figure 3: Neutrophil activation, autophagy and NETs are associated in the blood from SSc patients.
   A. Representative confocal microscopy of neutrophils from a patient with SSc showing the translocation of MPO from intracellular granules to the membrane.
   B. Neutrophil membrane MPO (mMPO) expression on neutrophils from SSc patients and matched healthy volunteers was quantified by flow cytometry. Each symbol refers to a single subject. ****, p< 0.0001 by unpaired t-test.
   C - D. Neutrophils from SSc patients and healthy volunteers were examined for autophagosomes (Cyto-ID) by confocal microscopy.
   E. Transmission electron microscopy of neutrophils from the blood of SSc patients reveals massive vacuolization and immature autophagic vacuoles.
   F. The fraction of Cyto-ID⁺ neutrophils in the blood of SSc patients and of healthy controls was assessed by flow cytometry. Each symbol refers to a single subject. Bars indicate mean±SEM. ****, p<0.0001 by unpaired t-test.
   G - H. The concentration of NET byproducts - soluble DNA-MPO complexes (G) and citrullinated H4 histone (H) - in the plasma of SSc patients and healthy volunteers. ****, p< 0.0001 by unpaired t-test.
   I. Correlation of citrullinated H4 and MPO-DNA complexes in plasma from SSc patients. Circles represent individual subjects.

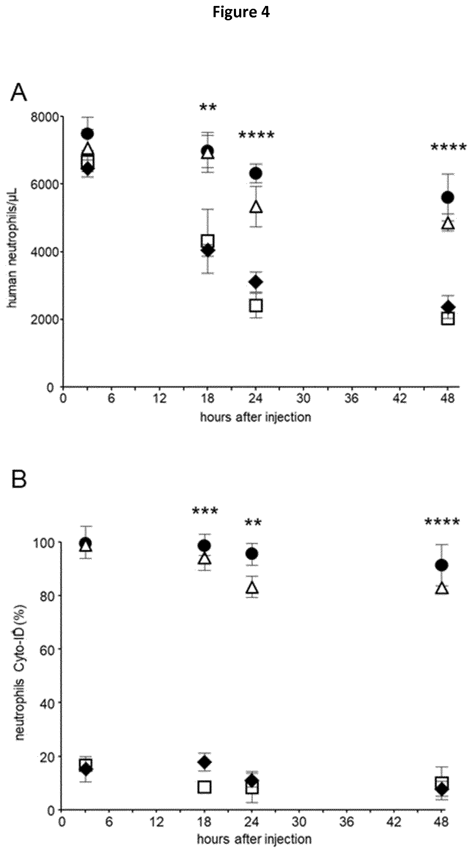

Figure 4: Interaction with SSc-µP prompts activation of autophagy and neutrophil survival.
   Human neutrophils from healthy donors were exposed (open triangles) or not (open squares) to SSc-µPs and injected in NSG mice. Human neutrophil counts in the mouse blood (y axis) were assessed at various times (x axis). A. Counts of SSc-µP-treated human neutrophils remained substantially and significantly higher than those of untreated neutrophils. The prolonged survival of neutrophils required autophagy, since it was abrogated by the blocker of the autophagic flux, wortmannin, added before the challenge with SSc-µPs (black diamonds). Moreover, the counts of human neutrophils committed to autophagy by treatment with rapamycin (black circles) and of SSc-µP-treated neutrophils were similar. B. The fraction of autophagic neutrophils at various times after injection (hours, x axis), as assessed based on the accumulation of Cyto-ID (y axis). SSc-µP-treated and rapamycin-treated neutrophils, whose survival was substantial (see graph A), were mostly autophagic. In contrast untreated and wortmannin-treated neutrophils, whose counts abated earlier, failed to undergo autophagy. Symbols indicates the mean and bars SEM of three different experiments conducted independently. ****, *** and **: significantly different from untreated neutrophils, p<0.0001, <0.001 and <0.01 respectively.

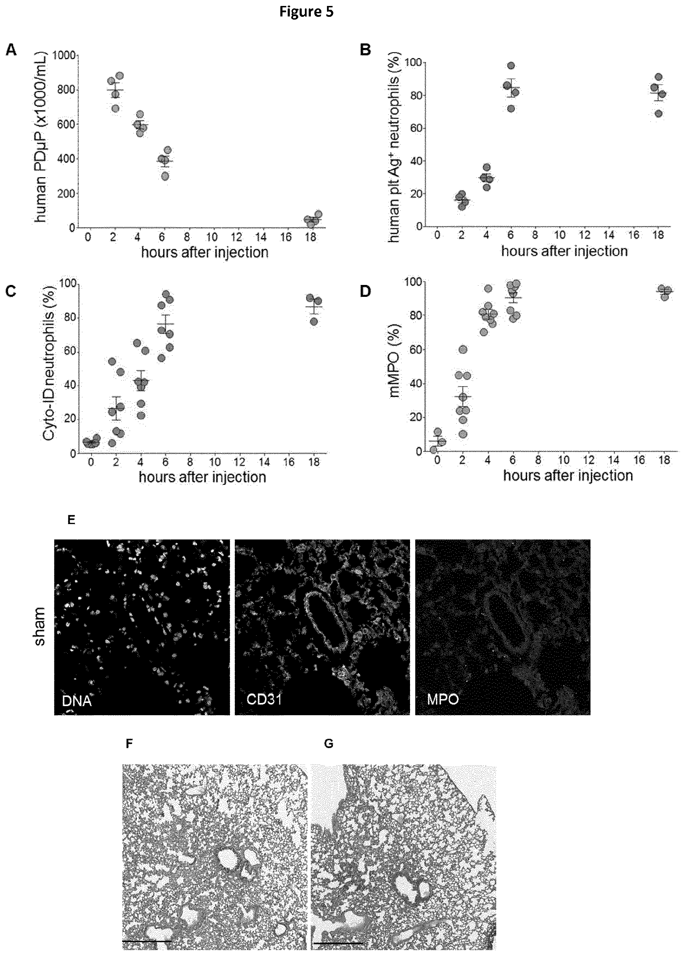

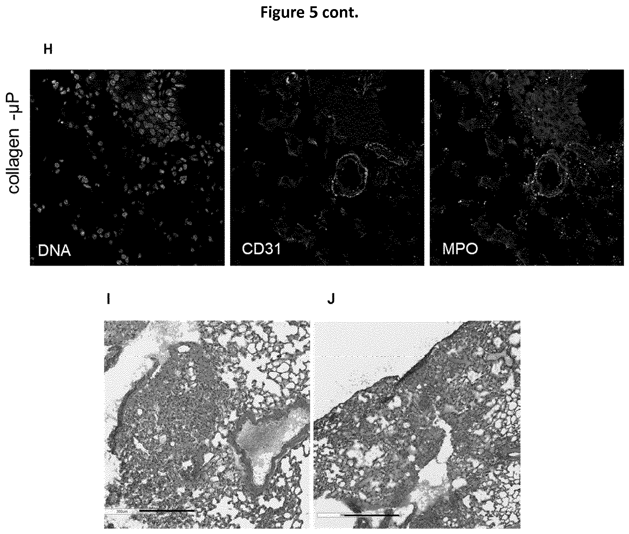

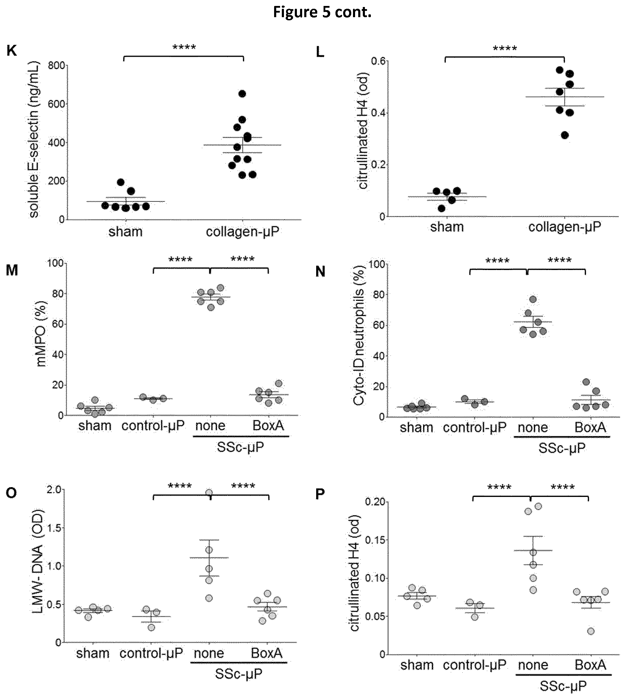

Figure 5: Platelet microparticles trigger neutrophil and endothelial activation, lung inflammation, and fibrosis in NSG mice.
   Human collagen-microparticles were injected into NSG mice and their blood counts assessed at various times based on the expression of the human CD61 (A). The fraction of murine neutrophils with adherent human microparticles (B), the fraction of autophagic neutrophils, identified based on the accumulation of Cyto-ID (C), and the fraction of neutrophils that expressed MPO on the plasma membrane (D) were assessed at various times after injection.
   The lungs of NSG mice were harvested 24 hours after injection with PBS (sham) (E-G) or with human collagen-microparticles (H-J). Sections were processed for confocal microscopy (E and H) and endothelial lining identified by CD31 staining, infiltrating neutrophils by MPO staining; nuclei were counterstained with Hoechst. Alternatively, sections were processed for histochemistry with hematoxylin and eosin (F and I) or Sirius red (G and J). Bars: 300 µm.
   Significantly increased concentrations of E-selectin (K) and of the NET byproduct, citrullinated H4 histone (I), assessed by ELISA in the plasma of mice injected with collagen-microparticles confirm systemic endothelial activation and NET generation, respectively. Microparticles from the plasma of patients with SSc (SSc-µP) or of healthy volunteers (control-µP) were injected into NSG mice in the presence or the absence of the HMGB1 inhibitor, BoxA. The fraction of murine neutrophils that expressed MPO on the plasma membrane (M) and the fraction of autophagic neutrophils (N) were assessed by flow cytometry, while the concentration in the plasma of low-molecular weight soluble DNA (O) or of citrullinated H4 histones (I) were assessed by ELISA. Symbols represent individual observations in each mouse injected with microparticles derived from a single donor. Lines represent the means ± SEM for each determination. ****, p<0.0001 unpaired t test (K and L), or ANOVA and Bonferroni's test (M-P).

### DETAILED DESCRIPTION OF THE INVENTION

### Definitions

The term "biomarker" means a distinctive biological or biologically derived indicator of a process, event, or condition. Biomarkers can be used in methods of diagnosis, e.g. clinical screening, assessment of severity or activity of disease and prognosis assessment and in monitoring the results of therapy, identifying patients most likely to respond to a particular therapeutic treatment, drug screening and development. Biomarkers and uses thereof are valuable for identification of new drug treatments and for discovery of new targets for drug treatment.

The term "diagnosis" as used herein encompasses identification, confirmation, and/or characterisation of systemic sclerosis. It includes assessment that the disease is in an active phase as opposed to an inactive phase. Efficient diagnosis and monitoring methods provide very powerful "patient solutions" with the potential for improved prognosis, by establishing the correct diagnosis and allowing rapid identification of the most appropriate treatment (thus lessening unnecessary exposure to harmful drug side effects).

The term "detecting" as used herein means confirming the presence of the biomarker present in the sample. Determining the level of the biomarker present in a sample may include determining the concentration of the biomarker present in the sample. Detecting and/or determining the level may be performed directly on the sample, or indirectly on an extract therefrom, or on a dilution thereof.

"Systemic sclerosis" is an autoimmune disease of the connective tissue, however, there is no clear obvious cause for it. The disease is characterized by thickening of the skin and of the connective tissue of visceral organs caused by accumulation of collagen, and by injuries to small arteries and capillaries. There are two forms of systemic sclerosis: cutaneous localized and systemic. The localized (limited) form affects the skin of only the face, hands, and feet. The systemic (diffuse) form involves more extensively the skin and, in addition, involves visceral organs, including the heart, lungs, and gastrointestinal tract.

"Systemic lupus erythematosus" (also known as lupus, or SLE) is an autoimmune disease in which the body's immune system mistakenly attacks healthy tissue in many parts of the body. Symptoms vary between people and may be mild to severe. Common symptoms include painful and swollen joints, fever, chest pain, hair loss, mouth ulcers, swollen lymph nodes, migraine, feeling tired, and a red rash which is most commonly on the face. Often there are periods of illness activity, called flares, and periods of remission during which there are few symptoms. The cause of lupus is not clear although it is thought to involve genetics together with environmental factors.

The term "biosensor" means anything capable of detecting the presence of the biomarker. Examples of biosensors are described herein.

The term "antibody" as used herein includes, but is not limited to: polyclonal, monoclonal, bispecific, humanised or chimeric antibodies, single chain antibodies, Fab fragments and F(ab')₂ fragments, fragments produced by a Fab expression library, anti-idiotypic (anti-Id) antibodies and epitope-binding fragments of any of the above. The term "antibody" as used herein also refers to immunoglobulin molecules and immunologically-active portions of immunoglobulin molecules, *i.e.,* molecules that contain an antigen binding site that specifically binds an epitope on an antigen. The immunoglobulin molecules of the invention can be of any class (e.g., IgG, IgE, IgM, IgD and IgA) or subclass of immunoglobulin molecule.

HMGB1 is a protein having the sequence of SEQ ID No. 1 in humans. The Box-A domain is a sequence having the DNA binding function of an HMG Box-A domain, comprising at least residues 23 to 45 of SEQ ID No 1 or the corresponding residues of a homologue thereof.

The Box-B domain is a sequence having the DNA binding function of an HMG Box-B domain comprising at least residues 115 to 145 of SEQ ID NO 1 or the corresponding residues of a homologue thereof.

There are homologues of HMGB1 in other species. An anti-HMGB1 antibody against the corresponding homologue can be used to diagnose and treat systemic sclerosis in non-human species.

The data described herein shows the development of a reproducible blood-based biomarker test, resulting from analysis of 57 patients classified according to the 2013 ACR/EULAR criteria for systemic sclerosis. It demonstrates that an increased level of platelet-derived microparticles expressing HMGB1 described herein, is statistically significant as a biomarker for the diagnosis of systemic sclerosis.

Accordingly, there is provided a method of diagnosing systemic sclerosis in an individual suspected of having systemic sclerosis, comprising:
(a) determining the level of platelet-derived microparticles expressing HMGB1 as a biomarker in a blood derived sample obtained from the individual;
(b) comparing the level of the biomarker in the blood derived sample with a control value, such that a higher level of the biomarker in the blood derived sample obtained from the individual as compared with the control value is indicative of a diagnosis of systemic sclerosis in the individual.

There is also provided a method of determining the severity of systemic sclerosis in a patient diagnosed with systemic sclerosis, comprising:
(a) determining the level of platelet-derived microparticles expressing HMGB1 as a biomarker in a blood derived sample obtained from the patient;
(b) comparing the level of the biomarker in the blood derived sample with a control value, such that the level of the biomarker in the blood derived sample obtained from the patient is higher than that of the control value, whereby the higher the level of the biomarker in the blood derived sample obtained from the patient as compared with the control value the greater the severity of the systemic sclerosis in the patient.

Detecting and/or determining the level can be performed by any method suitable to identify the presence and/or level of a specific microparticle in a blood derived sample from a patient or a purification or extract of a blood derived sample or a dilution thereof. In methods of the invention, determining the level may be performed by measuring the concentration of the biomarker in the sample or samples. For example, detecting and/or determining the level can be performed by a process comprising one or more method(s) selected from the group consisting of: flow cytometry, ELISA, FRET, BRET and light initiated chemiluminescent assay (LICA).

Determining the level of the biomarker may, for example, be performed by a process comprising use of anti-HMGB1 antibodies to bind to HMGB1 expressed by the microparticles.

In particular, determining the level of the biomarker is performed by a process comprising using anti-HMGB1 antibodies to bind to HMGB1 expressed by the microparticles and using another type of antibody which bind to another marker expressed by platelet derived microparticles; for example, the glycoproteins CD61, CD41 and CD42. For example, the other type of antibody binds to the human platelet antigen CD61 expressed by the platelet derived microparticles.

Thus, in uses and methods of diagnosing according to the invention, suitably the level platelet-derived microparticles expressing HMGB1 is determined by determining the level of HMGB1⁺CD61⁺ microparticles. Thus, the determining can be performed using antibodies which bind to HMGB1, or using antibodies which bind to HMGB1 and antibodies which bind to CD61. It may also be possible to use bispecific antibodies which bind to HMGB1 and CD61 or another platelet marker.

Suitably the antibodies are labelled, for example they are fluorescently labelled, and can be detected using techniques such as flow cytometry, and their concentration can be measured with fluorescence readers. As an example, a fluorescent label may emit fluorescence radiation of wavelength 525 to 700 nm when excited at 488 nm. Alternatively, antibodies may be labelled with another type of label that emits visible or invisible radiation.

In one particular example method, the level of the biomarker is determined by determining the level of microparticles to which both (a) anti-HMGB1 antibodies and (b) antibodies against another marker expressed by platelet derived microparticles have bound using a method based on the proximity of said antibodies (a) and (b). Such proximity may, for example, be measured as follows:
FRET (fluorescence resonance energy transfer) is a mechanism describing energy transfer between two light-sensitive molecules (chromophores). A donor chromophore, initially in its electronic excited state, may transfer energy to an acceptor chromophore through nonradiative dipole-dipole coupling. Measurements of FRET efficiency can be used to determine if two fluorophores are within a certain distance of each other. FRET is a common research tool in fields including biology and chemistry. Accordingly, when FRET is employed then the label on one type of antibody (binding HMGB1 or another platelet marker e.g. CD61) is a donor chromophore and the label on the other type of antibody is an acceptor chromophore. Proximity is detected by the emission of radiation at one wavelength after excitation of the donor chromophore with radiation of a different wavelength (appropriate for the donor chromophore).

BRET (bioluminescence resonance energy transfer) uses a bioluminescent luciferase (e.g. the luciferase from Renilla reniformis) to produce an initial photon emission, which is then captured by an acceptor chromophore; the rest of the assay is similar to a FRET assay.

A most effective method of detecting both HMGB1 and another antigen on the same microparticle is light initiated chemiluminescent assay (LICA). LICA is a chemiluminescence technology based on nanobeads providing homogenous, rapid, no-clean, quantitative and high-throughput test. The core principle of LICA is that irradiation causes photosensitized formation of singlet oxygen, which migrates to a bound particle and activates the chemiluminescer, thereby initiating a delayed luminescence emission. Photosensitive bead stimulated by red laser (680 nm) generates singlet oxygen, which will decay within about 4 microseconds. During its lifetime, the singlet oxygen passes the energy to the surrounding luminescent bead (usually within 200 nm of the luminescent microparticles), which combines with singlet oxygen and emits high-energy light (520-620 nm). Such delivery of chemical energy relying on two particles close to each other completes the cycle that provides stable signal pass and amplification of the signal.

Another assay based on proximity of two molecules, in this case HMGB1 and another antigen, is the proximity ligation assay (PLA). In a common embodiment of PLA, two primary antibodies raised in different species recognize the target antigens on the proteins of interest. Secondary antibodies directed against the constant regions of the different primary antibodies, called PLA probes, bind to the primary antibodies. Each of the PLA probes has a unique short DNA strand attached to it. If the PLA probes are in close proximity (e.g. they close to each other on a microparticle), the DNA strands can participate in rolling circle DNA synthesis when appropriate substrates and enzymes are added. The DNA synthesis reaction results in several-hundredfold amplification of the DNA circle. Next, fluorescent-labeled complementary oligonucleotide probes are added, and they bind to the amplified DNA. Fluoresce is thus proportional to the DNA produced in the assay.

Suitably, antibodies employed in methods, uses and kits of the invention are recombinant monoclonal antibodies. An example anti-HMGB1 antibody is the antibody of clone DHP1.1. An example antibody which binds CD61 is the antibody of clone SZ21.

Accordingly, there is provided a method of distinguishing systemic sclerosis from systemic lupus erythematosus in an individual, comprising:
(a) determining the level of platelet-derived microparticles expressing HMGB1 as a biomarker in a blood derived sample obtained from the individual;
(b) comparing the level of the biomarker in the blood derived sample with a control value, such that a higher level of the biomarker in the blood derived sample obtained from the individual as compared with the control value is indicative of a diagnosis of systemic sclerosis and not a diagnosis of SLE in the individual.

Patients diagnosed with systemic sclerosis can undergo a therapy regime which may include administering an anti-HMGB1 antibody to the patient. This is based on the observation that HMGB1-exposing microparticles can activate neutrophils and produce SSc-like features in mice, indicating that microparticle-associated HMGB1 is responsible for SSc pathogenesis. Accordingly, inhibiting HMGB1 might decrease SSc severity. The decision of administering an anti-HMGB1 antibody may be as a result of the patient having been identified as having increased levels of a platelet-derived microparticle expressing HMGB1 as a biomarker when compared to the levels of said biomarker when compared to a control value. Suitably the control value is from a normal individual.

Accordingly, there is provided a method of treatment of systemic sclerosis in a patient suffering from systemic sclerosis, which comprises the step of administering an effective amount of anti-HMGB1 antibody to said patient.

Suitably the patient for treatment has been identified as having an increased level of platelet-derived microparticles expressing HMGB1 as a biomarker in a blood-derived sample from said patient when compared to a control value

There is also provided an anti-HMGB1 antibody for use in the treatment of systemic sclerosis in a patient.

The antibody will be administered in an effective amount and at a frequency (e.g. once per week) which may be determined by a skilled person. The antibody will typically be administered parenterally e.g. intravenously or sub-cutaneously.

The anti-HMGB1 antibody should bind to platelet-derived microparticle expressing HMGB1 in the blood of patients with systemic sclerosis.

In therapeutic methods, the antibody may be provided in a formulation comprising the antibody and a pharmaceutically acceptable carrier. The carrier may, for example, be water or saline for injection. It may contain other pharmaceutically acceptable excipients selected from surfactants, tonicity modifying substances (such as sugars, salts) and preservatives.

There is also provided a method of treating a patient suffering from systemic sclerosis, which comprises the following steps:
(a) determining the level of platelet-derived microparticle expressing HMGB1 as a biomarker in a blood derived sample obtained from the patient
(b) comparing the level of the biomarker in the blood derived sample with a control value, whereby a higher level of the biomarker in the blood derived sample obtained from the patient as compared with the control value indicates a diagnosis of systemic sclerosis in the patient; and
(c) administering an anti-HMGB1 antibody to a patient diagnosed in step (b) as a patient with systemic sclerosis.

Suitably the method may further comprise determining the level of microparticles in the patient treated with an anti-HMGB1 antibody by using a secondary antibody against the anti-HMGB1 antibody. In this context, a secondary antibody is capable of binding to the anti-HMGB1 antibody when the latter antibody is bound to HMGB1. Thus, the secondary antibody may bind the Fc region of the anti-HMGB1 antibody. The secondary antibody may be labelled to allow it to be detected (e.g. labelled in the manner described elsewhere herein, such as with a fluorescent label)

There is also provided an anti-HMGB1 antibody for use in the treatment of systemic sclerosis in a patient who has been diagnosed with systemic sclerosis and has an increased level of platelet-derived microparticles expressing HMGB1 as a biomarker in a blood derived sample obtained from the patient as compared with the level of the biomarker in a blood derived sample obtained from a normal individual.

Suitably the anti-HMGB1 antibody is an antibody that binds immunospecifically to the Box A region of HMGB1 (i.e. the epitope of the antibody is within the BoX A region). Alternatively, the antibody is an antibody that binds immunospecifically to the Box B region of HMGB1 (i.e. the epitope of the antibody is within the Box B region).

In an embodiment, the anti-HMGB1 antibody is the antibody of clone DHP1.1 (available from HMGBiotech and other commercial providers).

During a therapy regime it may be such that the efficacy of the therapy in a patient is monitored in order to track the progress of the therapy and alter the regime as required. These monitoring methods can be incorporated into screens for new drug substances and combinations of substances.

As such, there is provided a method of assessing the efficacy of a therapy in a patient having or suspected of having active systemic sclerosis, comprising measuring at least two different time points, in a sample from said patient, the level of platelet-derived microparticles expressing HMGB1 as a biomarker whereby a reduction in the level of the biomarker with time indicates that the therapy is efficacious. The level of the biomarker in the sample may be compared with a control value at each time point.

It will be appreciated that references herein to a "reduction in the level" refer to a lower level of the biomarker in the test blood derived sample compared with the reference sample(s), which can be the level of the biomarker in the same patient before the therapeutic treatment.

In one embodiment, the lower level is a < 1 fold difference relative to the reference sample, such as a fold difference of 0.9, 0.8, 0.7, 0.6, 0.5, 0.4, 0.3, 0.2, 0.1, 0.05, 0.01 or any ranges therebetween. In one embodiment, the lower level is between a 0.1 and 0.85 fold difference relative to the reference sample, such as between a 0.2 and 0.7 fold difference relative to the reference sample. In a further embodiment, the lower level is between a 0.25 and 0.75 fold difference relative to the reference sample.

Monitoring methods of the invention can be used to monitor onset, progression, stabilisation, amelioration and/or remission.

In methods of diagnosing or monitoring according to the invention, detecting and/or determining the level of the biomarker in a blood derived sample from a test patient may be performed on two or more occasions. Comparisons may be made between the level of biomarker in samples taken on two or more occasions. Assessment of any change in the level of the biomarker in samples taken on two or more occasions may be performed. Modulation of the biomarker level is useful as an indicator of the state of the systemic sclerosis and its activity. An increase in the level of the biomarker, over time is indicative of onset or progression, *i.e.* worsening of this disorder, whereas a decrease in the level of the biomarker indicates amelioration or remission of the disorder.

A method of diagnosis of or monitoring according to the invention may comprise determining the level of the biomarker in a test blood derived sample from a test patient and comparing the level of the biomarker present in said test sample with a control value.

A higher level of the biomarker in the test sample relative to the level in the normal control is indicative of the presence of systemic sclerosis; an equivalent or lower level of the biomarker in the test sample relative to the normal control is indicative of an absence of systemic sclerosis. Higher levels of the biomarker indicate greater progression of systemic sclerosis than lower levels.

In a particular embodiment, there is provided a method of assessing the progression of systemic sclerosis in a patient suffering from systemic sclerosis, comprising:
(a) determining the level of platelet-derived microparticles expressing HMGB1 as a biomarker in a blood derived sample obtained from the patient;
(b) comparing the level of the biomarker in the blood derived sample obtained from the patient with a control value, such that the level of the biomarker in the blood derived sample obtained from the patient as compared with the control value is indicative of disease severity in the patient;
(c) comparing the level of the biomarker in the blood from the patient over time, so as to evaluate the extent or the rate of disease progression whereby an increase in the level of the biomarker in the blood derived sample obtained from the patient as compared with the control value is indicative of an increased disease severity in the patient and a reduction in the level of the biomarker in the blood derived sample obtained from the patient as compared with the control value is indicative of an reduced disease severity in the patient.

The "control value" used in a method of the invention can be the level of the biomarker found in a normal control sample from a normal individual expressed as one value (determined from one individual, or several individuals expressed as an average) or a range of values (determined from several individuals).

In one embodiment, the method comprises comparing the level of the biomarker in said test blood derived sample with the level known to be present in one or more samples taken from said patient prior to commencement of therapy, and/or one or more samples taken from said patient at an earlier stage of therapy.

Thus, in an embodiment, there is provided a method of assessing the efficacy of a therapy for systemic sclerosis in a patient suffering from systemic sclerosis, comprising:
(a) determining the level of platelet-derived microparticles expressing HMGB1 as a biomarker in a blood derived sample obtained from the patient;
(b) comparing the level of the biomarker in the blood derived sample with a control value, such that the level of the biomarker in the blood derived sample obtained from the individual as compared with the control value is indicative of disease severity in the patient;
(c) treating the patient with the therapy for systemic sclerosis;
(d) comparing the level of the biomarker in the blood from the patient after treatment, so as to evaluate the extent or the rate of disease progression whereby a reduction in the level of the biomarker in the blood derived sample obtained from the individual as compared with the control value is indicative of the therapy being efficacious in the treatment of systemic sclerosis in the patient.

Currently, there are no effective therapies for systemic sclerosis, partly because it is difficult to gauge disease progression based on clinical assessment only. Thus, the availability of a marker can be instrumental for the development of effective therapies for systemic sclerosis.

The method can also be used to determine the efficacy of a candidate drug in a drug trial (i.e. where "therapy" is understood to mean "potential therapy").

Suitably, the time elapsed between taking samples from a individual or patient undergoing diagnosis or monitoring will be 3 days, 5 days, a week, two weeks, a month, 2 months, 3 months, 6 or 12 months. Samples may be taken prior to and/or during and/or following therapy for systemic sclerosis, such as an antibody therapy. Samples can be taken at intervals over the remaining life, or a part thereof, of a individual.

Blood derived samples that may be tested in a method of the invention include whole blood, blood serum and plasma. Suitably the blood derived sample is a sample of plasma.

Biosensors according to the invention may comprise a ligand or ligands, as described herein, capable of specific binding to the biomarker. Such biosensors are useful in detecting and/or determining the level a biomarker of the invention.

Accordingly, there is provided the use of a kit comprising a biosensor capable of detecting and/or determining the level a platelet-derived microparticle expressing HMGB1 as a biomarker for monitoring or diagnosing systemic sclerosis in an individual.

The biomarker may be detected directly or indirectly via interaction with a ligand or ligands such as an antibody or a biomarker-binding fragment thereof, or other peptide, or ligand, e.g. aptamer, or oligonucleotide, capable of specifically binding the biomarker. The ligand may possess a detectable label, such as a luminescent, fluorescent or radioactive label, and/or an affinity tag.

The kit may, for example, comprise anti-HMGB1 and antibodies against another marker expressed by platelet-derived microparticle such as CD61.

The identification of key biomarkers specific to a disease is central to integration of diagnostic procedures and therapeutic regimes. Using predictive biomarkers, appropriate diagnostic tools such as biosensors can be developed, accordingly, in methods and uses of the invention, detecting and determining the level can be performed using a biosensor, microanalytical system, microengineered system, microseparation system, immunochromatography system or other suitable analytical devices. Using such biosensors, it is possible to detect the target biomarker at the anticipated concentrations found in blood derived samples.

Suitably the individual or patient of the methods, uses and kits of the invention is a human.

Further aspects of the invention are defined by the following clauses:
Clause 1. Use of platelet-derived microparticles expressing HMGB1 as a biomarker for the diagnosis of systemic sclerosis, assessment of severity and progression of systemic sclerosis and assessment of the efficacy of therapy for systemic sclerosis.
Clause 2. A method of diagnosing systemic sclerosis in an individual suspected of having systemic sclerosis, comprising:
   (a) determining the level of platelet-derived microparticles expressing HMGB1 as a biomarker in a blood derived sample obtained from the individual;
   (b) comparing the level of the biomarker in the blood derived sample with a control value, such that a higher level of the biomarker in the blood derived sample obtained from the individual as compared with the control value is indicative of a diagnosis of systemic sclerosis in the individual.
Clause 3. A method of determining the severity of systemic sclerosis in a patient diagnosed with systemic sclerosis, comprising:
   (a) determining the level of platelet-derived microparticles expressing HMGB1 as a biomarker in a blood derived sample obtained from the patient;
   (b) comparing the level of the biomarker in the blood derived sample with a control value, such that the level of the biomarker in the blood derived sample obtained from the patient is higher than that of the control value, whereby the higher the level of the biomarker in the blood derived sample obtained from the patient as compared with the control value the greater the severity of the systemic sclerosis in the patient.
Clause 4. A method of assessing the progression of systemic sclerosis in a patient suffering from systemic sclerosis, comprising:
   (a) determining the level of platelet-derived microparticles expressing HMGB1 as a biomarker in a blood derived sample obtained from the patient;
   (b) comparing the level of the biomarker in the blood derived sample obtained from the patient with a control value, such that the level of the biomarker in the blood derived sample obtained from the patient as compared with the control value is indicative of disease severity in the patient;
   (c) comparing the level of the biomarker in the blood from the patient over time, so as to evaluate the extent or the rate of disease progression whereby an increase in the level of the biomarker in the blood derived sample obtained from the patient as compared with the control value is indicative of an increased disease severity in the patient and a reduction in the level of the biomarker in the blood derived sample obtained from the patient as compared with the control value is indicative of an reduced disease severity in the patient.
Clause 5. A method of assessing the efficacy of a therapy for systemic sclerosis in a patient suffering from systemic sclerosis, comprising:
   (a) determining the level of platelet-derived microparticles expressing HMGB1 as a biomarker in a blood derived sample obtained from the patient;
   (b) comparing the level of the biomarker in the blood derived sample with a control value, such that the level of the biomarker in the blood derived sample obtained from the individual as compared with the control value is indicative of disease severity in the patient;
   (c) treating the patient with the therapy for systemic sclerosis;
   (d) comparing the level of the biomarker in the blood from the patient after treatment, so as to evaluate the extent or the rate of disease progression whereby a reduction in the level of the biomarker in the blood derived sample obtained from the individual as compared with the control value is indicative of the therapy being efficacious in the treatment of systemic sclerosis in the patient.
Clause 6. A method of assessing the efficacy of a therapy in a patient having or suspected of having systemic sclerosis, comprising measuring at at least two different time points, in a sample from said patient, the level of platelet-derived microparticles expressing HMGB1 as a biomarker whereby a reduction in the level of the biomarker with time indicates that the therapy is efficacious.
Clause 7. The method according to clause 6, comprising comparing the level of the biomarker present in said test sample with a control value at each time point.
Clause 8. The method according to clause 6 or clause 7 comprising comparing the level of the biomarker in said test sample with the level in one or more samples taken from said patient prior to commencement of therapy, and/or one or more samples taken from said patient at an earlier stage of therapy.
Clause 9. The method according to any of clauses 6 to 8, wherein samples are taken prior to and/or during and/or following therapy for systemic sclerosis.
Clause 10. The method according to any of clauses 2 to9, wherein determining the level of the biomarker is performed by a process comprising flow cytometry.
Clause 11. The method according to any one of clauses 2 to 10 wherein determining the level of the biomarker is performed by a process comprising use of anti-HMGB1 antibodies to bind to HMGB1 expressed by the microparticles.
Clause 12. The method according to clause 11 wherein the determining the level of the biomarker further comprises the use of a secondary antibody against the anti-HMGB1 antibodies.
Clause 13. The method according to clause 11 wherein determining the level of the biomarker is performed by a process comprising using anti-HMGB1 antibodies to bind to HMGB1 expressed by the microparticles and using another type of antibody which bind to another marker expressed by platelet derived microparticles.
Clause 14. The method according to clause 13 wherein the other type of antibody binds to CD61 expressed by the platelet derived microparticles.
Clause 15. The method according to any one of clauses 12 to 14 wherein the anti-HMGB1 antibodies and the other type of antibody are labelled.
Clause 16. The method according to clause 15 where the label is a fluorescent label or another type of label that emits visible or invisible radiation.
Clause 17. The method according to clause 13 or clause 16 wherein the level of the biomarker is determined by determining the level of microparticles to which both (a) anti-HMGB1 antibodies and (b) antibodies against another marker expressed by platelet derived microparticles have bound using a method based on the proximity of said antibodies (a) and (b).
Clause 18. The method according to clause 17 wherein the method based on the proximity of said two types of antibodies is FRET or BRET, LICA or PLA.
Clause 19. The method according to any of clauses 3 to 18, wherein the blood derived sample is a sample of whole blood, blood serum or plasma.
Clause 20. Use of a kit comprising a biosensor capable of detecting and/or determining the level of a platelet-derived microparticle expressing HMGB1 as a biomarker for monitoring or diagnosing systemic sclerosis in an individual.
Clause 21. A method of treatment of systemic sclerosis in a patient suffering from systemic sclerosis, which comprises the step of administering an effective amount of anti-HMGB1 antibody to said patient.
Clause 22. An anti-HMGB1 antibody for use in the treatment of systemic sclerosis in a patient.
Clause 23. The method or antibody for use according to clause 21 or 22 wherein the patient has been identified as having an increased level of platelet-derived microparticles expressing HMGB1 as a biomarker in a blood-derived sample from said patient when compared to a control value.
Clause 24. A method of treating a patient suffering from systemic sclerosis, which comprises the following steps:
   (a) determining the level of platelet-derived microparticles expressing HMGB1 as a biomarker in a blood derived sample obtained from the patient;
   (b) comparing the level of the biomarker in the blood derived sample with a control value, whereby a higher level of the biomarker in the blood derived sample obtained from the patient as compared with the control value indicates a diagnosis of systemic sclerosis in the patient; and
   (c) administering an anti-HMGB1 antibody to a patient diagnosed in step (b) as a patient with systemic sclerosis.
Clause 25. The method according to any one of clauses 21, 23 or 24 which further comprises determining the level of platelet-derived microparticles expressing HMGB1 in a blood-derived sample obtained from a patient treated with an anti-HMGB1 antibody by using a secondary antibody against the said anti-HMGB1 antibody.
Clause 26. An anti-HMGB1 antibody for use in the treatment of systemic sclerosis in a patient who has been diagnosed with systemic sclerosis and has an increased level of platelet-derived microparticles expressing HMGB1 as a biomarker in a blood derived sample obtained from the patient as compared with the level of the biomarker in a blood derived sample obtained from a normal individual.
Clause 27. The method, use or antibody for use according to any one of clauses 11 to 26 wherein the anti-HMGB1 antibody is the antibody of clone DHP1.1.
Clause 28. The method, use or antibody for use according to any one of clauses 11 to 26 wherein the anti-HMGB1 antibody binds immunospecifically to the Box A region of HMGB1.
Clause 29. The method, use or antibody for use according to any one of clauses 11 to 26 wherein the anti-HMGB1 antibody binds immunospecifically to the Box B region of HMGB1.

### EXAMPLES

The study group consisted of 57 patients classified according to the 2013 ACR/EULAR criteria for SSc. Patients with other systemic autoimmune disorders or overlap syndromes were excluded. 53 age-matched healthy donors (median 52 years old, range 25-74; 36 females, 17 males) and six patients fulfilling ≥4 of the 1982 ACR SLE classification criteria served as controls. **Table 1** summarizes some demographic and clinical characteristics of patients with SSc. The pattern at nailfold videocapillaroscopy (NVC) was classified and categorized as early, active, and late as described. All patients and controls gave their written informed consent to participate to the study. The Institutional Review Board approved the study and the bio banking of blood derivatives.

**Table 1: SSc patients' characteristics**

| | |
|---|---|
| N | 57 |
| Sex, male/female | 11/46 |
| Age, median (range) years | 54 (33-80) |
| History of cardiovascular disease (n) | 8 |
| History of neoplasy (n) | 0 |
| Disease duration, median (range) years | 4 (1-31) |
| Disease subset: diffuse/limited | 25/32 |
| Sicca syndrome (n) | 15 |
| modified Rodnan skin score, median (range) | 5 (0-41) |
| NVC (scleroderma pattern/unspecific findings) | 46/11 |
| Fingertip ulcers (n) | 26 |

| Pulmonary involvement | |
|---|---|
| Pulmonary fibrosis (n) | 15 |
| Pulmonary hypertension (n) | 4 |
| Arterial pulmonary hypertension (n) | 1 |

| Autoantibodies | |
|---|---|
| Anti-nuclear antibodies (n) | 57 |
| Anti-topoisomerase I antibodies (n) | 24 |
| Anti-centromere antibodies (n) | 30 |

| Treatment | |
|---|---|
| Aspirin (n) | 33 |
| Tienopiridine (n) | 4 |
| Hydroxychloroquine (n) | 11 |
| Prednisone (n) | 12 |
| Dose median (range) | 5 (2.5-7.5) |
| Immune suppressive drugs (n) | 12 |
| Bosentan (n) | 6 |

### Methods and materials

**Reagents and monoclonal antibodies** (mAbs). Thrombofix, Flow-Count™ Fluorospheres, mAbs against CD61 (clone SZ21), CD66b (clone 80H3), myeloperoxidase (MPO, clone CLB-MPO-1), CD42 (platelet glycoprotein Ib, SZ1), CD62P (P-selectin; Thromb-6), relevant IgG isotype control mAbs, the Annexin V/ 7-AAD kit and Flow-Count™ Fluorospheres were obtained from Beckman Coulter (Italy). Monoclonal mAb against HMGB1 (clone HAP-46.5), PGE1, rapamycin, wortmannin, Cell Death detection kit and Cell Death detection kit PLUS, Sirius Red and Hoechst were obtained from Sigma (Italy). FACs Lysing Solution and mAbs against murine CD45 (clone 30-F11) were obtained from Beckton Dickinson. Collagen and the ELISA kit for E-selectin were obtained from Horm (Cabru, Italy). Zenon IgG Labeling kits (488 or 546) were obtained from Invitrogen. The CYTO-ID® Autophagy detection kit was obtained by Enzo Life Sciences (3v Chimica, Milan, Italy). Monoclonal antibodies used to identify murine neutrophils (clone 7/4), rabbit polyclonal anti-histone H4 (citrulline R3, ab81797) and mAb anti RAGE (ab54741) antibodies were obtained from Abcam (Prodotti Gianni, Italy). Liposomes containing clodronate and liposomes containing PBS were obtained from ClodronateLiposomes.org (The Netherlands). mAb anti human MPO used for the determination of DNA-MPO complexes was from ABD Serotec. mAb against activated Mac-1(clone CBRM1/5) was obtained from Biolegend. mAb anti LC3 (biorbyt, clone 5H12) was obtained from DBA. BoxA (amino acids 2 to 89 of SEQ ID No.1) and recombinant HMGB1 were obtained from HMGBiotech (Milan).

**Blood sampling.** Venous blood was drawn through a 19-gauge butterfly needle. After having discarded the first 3-5 mL, blood was carefully collected in tubes containing either EDTA to purify microparticles and to assess the concentration of NETs byproducts, or Na citrate and antiproteases for flow cytometry or Na-citrate + EDTA for neutrophil and platelet isolation.

**Flow cytometry.** Blood samples (anticoagulated with citrate + antiproteases) were immediately fixed with equal volumes of Thrombofix and analyzed on a Navios flow cytometer (Beckman Coulter, Milan, Italy). Extent of platelet and leukocyte activation, and of HMGB1 expression and autophagy were assessed by multi parametric flow cytometry as previously described (Maugeri et al., 2012). Quantification and characterization of PD-microparticles was performed in platelet- free plasma samples or collagen-microparticle samples.

**Human cells and microparticle.** Platelets and neutrophils were purified from the peripheral blood and characterized as described (Evangelista V et al, Blood 1999; 93:876-885). Microparticles from SSc patients, SLE patients and healthy control subjects were isolated from EDTA anticoagulated plasma and characterized as described (Manfredi et al., 2016).

**Collagen-microparticles.** Purified platelets (5x10⁵/µL) were stimulated with collagen (5 µg/mL) for 5-60 minutes at 37°C or with Thrombin Receptor Agonist Peptide 6 (TRAP-6; 15 µM) in static conditions, placed in chilled water for 2 min and centrifuged (15 min, 2,000 g, 4°C). Supernatants were retrieved, centrifuged again (1 min, 100,000g, 4°C) aliquoted and frozen. Immediately before use, thawed aliquots were centrifuged for 60 min at 100,000g at 4°C, pellets resuspended in Hepes Tyrode buffer, and microparticles characterized and quantified by flow cytometry **(****Figure 1** and **Table 3).**

**Autophagy.** Neutrophil autophagy was determined by flow cytometry and confocal microscopy using CYTO-ID® kit according to the manufacturer instructions or by electron microscopy as previously described (Manfredi et al., 2016). Neutrophil autophagy assessment by Cyto-ID® dye incorporation into autophagosomes and by anti-LC3 antibodies was found to yield similar results. Cyto-ID® immunofluorescence is independent of the permeabilization steps necessary for the determination of LC3 and as such is better when studying adherence to microparticles together with induction of autophagy.

**Human or murine NETs generated *in vitro.*** NETs were generated as previously described (Manfredi et al., 2016). Briefly, neutrophils (5x10⁶/mL) were placed on poly-L-lysine-coated slides for 20 min at 37°C and challenged or not with soluble recombinant HMGB1 (10 µg/mL) or with microparticles (collagen-, SSc- or control-), at a microparticles:neutrophil ratio = 20:1. When indicated, microparticles were pre-treated with BoxA (10 µg/mL). After 20 min, plates were centrifuged (5 min, 1000g at 4°C), supernatants retrieved, further cleared by centrifugation (5 min, 100,000g at 4°C) and frozen until further analysis. Cell-free soluble DNA-MPO complexes, citrullinated histone H4 and low molecular weight DNA were assessed in the culture supernatant or in the plasma by ELISA. Slides were fixed with Thrombofix and placed at 4°C for 18-48 hours until confocal microscopy analysis.

**NETs quantification.** MPO-DNA complexes were identified using a capture ELISA as previously described (Manfredi et al., 2016) with minor modifications. Multiwell plates were coated with antihuman MPO mAb (capture) overnight at 4°C and blocked in 1% BSA. After washing human platelet-free plasma or culture supernatants were incubated with peroxidase-conjugated anti-DNA antibodies (clone MCA-33, from the cell death detection ELISA kit), incubated two hours at room temperature, washed and the reaction was developed with the peroxidase substrate (ABTS) of the kit. Citrullinated histone H4 was identified using a capture ELISA. In this case multiwell plates were coated with polyclonal antibody against citrullinated H4 (capturing antibody) overnight at 4°C and blocking in 1% BSA. After washing, platelet-free plasma or culture supernatants were placed in combination with the peroxidase-conjugated anti-DNA antibodies and then processed as above. Mono-Oligo nucleosomes (low molecular weight DNA) were assessed in murine plasma samples using the commercial cell death detection ELISA kit^{Plus} according to the manufacturer's instructions. Absorbance was assessed at 405 nm and 490 nm as reference wavelength.

**Proteolytic activity.** The proteolytic capacity of neutrophils was assessed using a fibrin plate assay as described (Ilich et al., 2017). Samples containing 5,000 neutrophils stimulated or not with collagen-, control- or SSc-microparticles were incubated 24 hrs at 37°C. The area of fibrin dissolution was quantified and expressed as mm².

**Mice.** Eight- to ten-week-old male NOD.Cg-Prkdcscid Il2rgtm1Wjl/SzJ mice (NSG) were provided by Attilio Bondanza (Milano). Mouse experiments were performed in accordance with national and institutional guidelines and all experiments were approved by the Institutional Animal Care and Use Committee of the San Raffaele Scientific Institute (IACUC-684). In preliminary experiments, neutrophils were isolated from the bone marrow of wild-type and of NSG mice, stimulated in vitro and compared for the ability to respond to various stimuli. Collagen-, SSc- or control-microparticles (10⁷ microparticles in PBS, 100 µL/mouse) were injected in the tail vein of NSG mice. Sham-treated mice were injected with PBS (100 µL). At different times, blood was obtained from the orbital vein of the mice and processed to determine NETs byproducts or the presence of human platelet antigens and neutrophil activation by flow cytometry. To define the requirement for murine neutrophils, control liposomes (PBS) or clodronate liposomes were injected i.v., in order to eliminate neutrophils in circulation. After 16 hours, collagen-microparticles or PBS were injected i.v. and blood samples obtained after 24 hours and processed to determine neutrophil viability, activation and in vivo NET formation. Lungs were isolated, fixed and analyzed to determine inflammatory infiltration and tissue damage. In separate sets of experiments, neutrophils purified from healthy volunteers as described above were challenged with SSc-microparticles at a 20:1 microparticle:neutrophil *ratio* for 15 min at room temperature in the presence or the absence of wortmannin (0.05 µM), washed and injected in the tail vein (9±1x10⁶ human neutrophils/NSG mouse). Control mice were injected with human neutrophils either untreated or treated with rapamycin (100 nM) and identically processed. Blood was retrieved at different times after injection and the concentration, the viability and the autophagy of injected neutrophils were assessed by flow cytometry after tagging with mAbs against the human antigens, CD45 and CD66b. Autophagy was determined by flow cytometry. To assess the clearance of the injected human neutrophils, phagocytosed human neutrophils were traced in the mouse spleen. Briefly, whole spleens were placed in C Tubes and dissociated by using the gentleMACS™ Dissociator system as indicated by the manufacturer. Cellular suspensions were washed twice with ice-cold PBS and then fixed with the solution A of the Fix and Perm Kit. Then, samples were labelled with mAbs against murine macrophages (F4/80), permeabilized (or not) with the solution B of the kit and labelled with mAbs against human neutrophils (CD66b) and analyzed and counted by flow cytometry as above.

**Histochemistry.** Lungs were isolated and immediately fixed in fresh 4% paraformaldehyde at 4°C for 6 hrs. For better cryopreservation, tissue were placed in 10%, 20% and 30% sucrose (each step with 24 hrs of incubation) tissue sinks embedded in optical cutting temperature (OCT) compound and stored at -80°C. 10 µm thick lung slices were prepared for histology assay.

**Statistical analysis.** Results were reported as mean ± standard error of the mean (SEM), unless otherwise indicated. The normal distribution of each continuous variable was assessed with the use of the Kolmogorov-Smirnov test. All patient data sets were found to be normally distributed. Statistical analysis were performed by one way - ANOVA, followed by Bonferroni Multiple Comparison Test or Student t test for comparison of two different groups. All tests were two-sided and *p* values lower than 0.05 were considered statistically significant. In all analyses GraphPad Prism 5.00 was used.

### Results

### Result 1 - Microparticles in the blood of SSc patients express HMGB1

Antibodies against CD61 were used to identify platelets and platelet-derived microparticles by flow cytometry in whole blood from healthy controls and patients with SSc or SLE. Platelet-derived microparticles, identified by the expression of the platelet-specific CD61 antigen, were more abundant in both whole blood and platelet-free plasma from SSc patients than from healthy volunteers or from patients with SLE **(****Figure 1****,** panels **A-B**). Microparticles of SSc patients expressed higher levels of HMGB1 than microparticles of healthy volunteers or of SLE patients, as assessed by flow cytometry **(****Figure 1****,** panel **B**). The concentration of platelet-derived CD61⁺ microparticles and of CD61⁺ microparticles expressing HMGB1 (calculated using Flow-Count™ Fluorospheres). HMGB1⁺CD61⁺ microparticles in particular were vastly more numerous in patients with SSc than in patients with SLE or in healthy controls (**Figure 1****,** panel **B** and **Table 2**). Purified platelets from healthy controls, activated with physiological stimuli (collagen or the thrombin receptor agonist, TRAP-6) exposed P-selectin (**Figure 1****,** panel **C**) and in a time-dependent manner released microparticles that expressed CD61, CD42 and HMGB1, as assessed by flow cytometry. They poorly expressed P-selectin (CD62P) and tissue factor (CD142) (**Figure 1****,** panel **C**). In keeping with the scope of our work we chose collagen, a constituent of the sub-endothelial layers of injured vessels, to commit platelets to the generation of microparticles (collagen-microparticles). Complexity, as assessed by side scatter, was similar in collagen-microparticles and SSc-microparticles (10±2 and 11±2 mean log side scatter, respectively).

**Table 2: Microparticles from the blood of SSc patients triggers neutrophils and endothelial cell activation**

| | **Healthy controls** | **SSc** | **SLE** |
|---|---|---|---|
| N | 53 | 57 | 5 |
| Leukocytes/µL | 5793±106 | 6922±458* | 5933.3±113 |
| Neutrophils /µL | 3546±83 | 4663±536 | 3500±543 |
| Platelets x10³ /µL | 200±5 | 223±17 | 248±5 |

| *A: whole blood* | | | |
|---|---|---|---|
| CD66b⁺CD16⁻ neutrophils (%) | 1.0±0.1 | 3.6±1.2 | 21.6±10.9*^{#} |
| Cyto-ID⁺ neutrophils (%) | 4.3±1 | 78.9±3.9* | 33.4±6.4*^{#} |
| P-selectin⁺ platelets (%) | 6.2±0.3 | 20.9±1.6* | 20.1±4.8* |
| TF⁺ platelets (%) | 4.9±0.6 | 22.3±3.8* | 26.4±6.6* |

| *B: platelet-free plasma* | | | |
|---|---|---|---|
| MPO-DNA complexes (a.u.) | 0.2±0.03 | 1.2±0.08* | 1.1±0.39* |
| Platelet (CD61⁺) µPx10³/µL | 19.1±4.8 | 184.4±19.3* | 24.4±11.4 |
| Platelet (CD61⁺) HMGB1⁺ µP x10³/µL | 2.9±0.3 | 133.4±13.8* | 4.7±1.0^{#} |

| *C: in vivo effects elicited by control-, SSC- or SLE-microparticles* | | | |
|---|---|---|---|
| Autophagic murine neutrophils (%) | 6.5±0.6 | 62.3±3.6* | 23.5±6.5^{#5} |
| mMPO murine neutrophils (%) | 11.0±0.6 | 77.8±2.1* | 17.7±3.9^{#} |
| Citrullinated H4 (a.u.) | 0.06±0.01 | 0.14±0.01* | 0.07±0.02^{#} |
| Soluble E-selectin (ng/mL) | 10.7±2.1 | 223.2±74.6* | 13.9±1.2^{#} |

### Result 2 - SSc microparticles prompt healthy neutrophils to redistribute intracellular granule content, to undergo autophagy and to generate NETs via HMGB1

The bioactivity of microparticles was verified by adding them to neutrophils freshly purified from the blood of healthy donors. Neutrophils were stimulated or not with collagen-microparticles, and membrane MPO expression (mMPO; representative of ten experiments carried out independently) was revealed by confocal microscopy and flow cytometry. Dependent on the concentration of microparticles they were exposed to, neutrophils redistributed their granular content and increased the expression of MPO on their plasma membrane (**Figure 2****,** panel **A**). The fraction of neutrophils expressing MPO on the surface also increased in a dose-dependent manner (**Figure 2****,** panel **A**). microparticles -challenged neutrophils in parallel acquired the ability to proteolyse fibrin, suggesting that proteinases redistributed at the plasma membrane were biologically active. The extent of the fibrinolytic action was dependent on the dose of HMGB1⁺ microparticles used to challenge neutrophils (**Figure 2****,** panel **A**). Both collagen- and SSc-microparticles were effective (**Figure 2****,** panel **A**). In contrast, neutrophils challenged with microparticles purified from healthy controls and untreated neutrophils degraded fibrin at a similar low extent. Recombinant, soluble HMGB1 also activated neutrophils to degrade fibrin (**Figure 2****,** panel **A**).

HMGB1 is a known inducer of autophagy, a process by which stressed cells ingest effete organelles and protein aggregates to provide anabolic substrates to feed their own bioenergetics. We thus purified healthy neutrophils and verified whether they underwent autophagy upon interaction with HMGB1⁺ microparticles *in vitro.* Both SSc- and collagen-microparticles induced autophagy, as assessed by flow cytometry monitoring the intracellular accumulation of the CYTO-ID® dye, which selectively labels autophagic vacuoles (**Figure 2****,** panel **B**). Control-microparticles only marginally influenced neutrophils. Autophagy depended on HMGB1, since SSc- and collagen- microparticles failed to influence neutrophils in the presence of the HMGB1 competitive antagonist, BoxA (**Figure 2****,** panel **B**).

Adherent neutrophils rely on autophagy to extrude decondensed chromatin threads containing citrullinated histones, referred to as NETs. Indeed, SSc- and collagen-microparticles induced both autophagy and NET generation. Microparticles expressing the CD42 platelet antigen (red) were often found associated to neutrophils producing NETs (**Figure 2****,** panel **C**) and soluble MPO/DNA complexes accumulated in the culture supernatant, as assessed by ELISA (**Figure 2****,** panel **C**). Control-microparticles did not detectably induce NET generation. NET generation abated in the presence of BoxA (**Figure 2****,** panel **C**). Conversely, as expected, adherent neutrophils challenged with recombinant human HMGB1 underwent autophagy and released NETs (**Figure 2****,** panel **C**). The interference with the HMGB1/RAGE axis virtually abrogated the induction of autophagy and the generation of NETs (**Table 3**).

**Table 3: Cellular adhesion molecules ligand pairs potentially involved in microparticle/neutrophil interaction**

| | Blocking mAbs against | Cyto-ID % | MPO-DNA complexes (OD) |
|---|---|---|---|
| Neutrophils alone | n/a | 15.8±1.7 | 0.35±0.12 |
| Neutrophils + Coll-microparticles | irrelevant Ag | 81.3±1.0 | 1.28±0.46 |
| | P-selectin | 79.3±2.2 | 1.23±0.48 |
| | PSGL1 | 80.5±1.0 | 1.37±0.53 |
| | CD42 | 78.5±1.5 | 1.24±0.49 |
| | Activated Mac-1 | 82.8±4.0 | 1.21±0.43 |
| | HMGB1 | 15.0±1.2 | 0.35±0.15 |
| | RAGE | 21.5±1.4 | 0.41±0.12 |

### Result 3 - Neutrophil autophagy and NETs in the blood of SSc patients

Neutrophils of patients with SSc express MPO at the plasma membrane as assessed by confocal microscopy. Nuclei were counterstained with Hoechst. Neutrophils of SSc patients appeared to be primed having translocated granular enzymes at the plasma membrane (**Figure 3****,** panels **A** and **B**). Moreover, blood neutrophils of patients with SSc, but not of healthy volunteers, exhibited features of autophagy as assessed by confocal microscopy (**Figure 3****,** compare Cyto-ID® accumulation in autophagosomes of healthy neutrophils in panel C and in SSc neutrophils in panel **D**) and confirmed by electron microscopy (**Figure 3****,** panel **E**). The fraction of autophagic blood neutrophils as assessed by flow cytometry was significantly higher in SSc patients than in controls (**Figure 3****,** panel **F,** *p*<0.0001). Markers of systemic inflammation, extent of cutaneous and pulmonary fibrosis or any other clinical feature of the patients shown in **Table 1** did not correlate with the fraction of circulating neutrophils that accumulated the Cyto-ID® dye.

Platelet-derived HMGB1⁺ microparticles also influence neutrophils' ability to generate NETs. The inventors found that soluble DNA-MPO complexes or citrullinated H4 histones were significantly more concentrated in the plasma of SSc patients than of healthy controls (both p < 0.0001; **Figure 3****,** panels **G-H**). The correlation between the concentration of citrullinated H4 histones and of MPO-DNA complexes (r=0.70, *p* <0.0001; **Figure 3****,** panel **I**) suggests that they reflect the same biological event, *i.e.* NET generation in SSc patients. MPO-DNA complexes were significantly more concentrated in the plasma of patients with an early and active scleroderma pattern compared with those with a late scleroderma pattern at the high magnification assessment by nailfold videocapillaroscopy (NVC) (1.13±0.08 vs 0.67±0.02 au, *p* < 0.002), suggesting that this approach reveals characteristics of the patients' microvasculature that correlate with the accumulation of NET byproducts.

### Result 4 - Platelet-derived microparticles prompt autophagy and survival of human neutrophils in NSG mice

To test if microparticles could extend the survival of neutrophils *via* apoptosis inhibition, the inventors exposed healthy human neutrophils to SSc- microparticles, in the presence or the absence of the blocker of the autophagic flux, wortmannin, and injected them in NSG mice. NSG mice have a virtually complete defect in adaptive immunity and are thus highly receptive to engraftment of human cells and tissues. Neutrophils either left untreated or committed to autophagy by treatment with rapamycin served as controls. Three hours after injection there was no difference in the blood counts of human neutrophils, regardless of previous treatments (**Figure 4****,** panel **A**). Later, the counts of SSc-microparticle neutrophils were significantly higher than those of untreated neutrophils (**Figure 4****,** panel **A**). SSc- microparticle neutrophils at these time points were mostly autophagic (**Figure 4****,** panel B). Autophagy induction by rapamycin had a similar effect, prompting both autophagy and survival (**Figure 4****,** panels **A** and **B**). Autophagy inhibition with wortmannin abrogated the extended survival in the circulation of SSc- microparticle-activated neutrophils (**Figure 4****,** panels **A** and **B**), suggesting that autophagy and survival are causally linked.

### Result 5 - Scleroderma microparticles prompt neutrophil activation and NET generation in NSG mice

To further explore the pathophysiological relevance of neutrophil activation and NET generation the inventors injected purified microparticles intravenously in NSG mice and traced them using antibodies selectively recognizing the human platelet antigen CD61. The fraction of murine neutrophils with adherent human microparticles, the fraction of autophagic neutrophils were identified based on the accumulation of Cyto-ID and the fraction of neutrophils that expressed MPO on the plasma membrane was assessed at various times after injection. The lung of NSG mice was retrieved after injection with PBS or with human collagen-microparticles. Sections were processed for confocal microscopy and endothelial linings identified based on expression of CD31, infiltrating neutrophils on the expression of MPO while nuclei were counterstained with Hoechst. Alternatively, sections were processed for histochemistry with hematoxylin and eosin or Sirius red. Representative photomicrographs of lung sections from mice injected with microparticles reveal infiltration with inflammatory cells (I) and collagen accumulation. The concentration of human microparticles was high two hours after injection (798,800±85,000 /mL) and progressively decreased at later times (**Figure 5****, panel A**). In parallel, the fraction of mouse neutrophils with adherent microparticles increased (**Figure 5****, panel B**). Most blood neutrophils accumulated the Cyto-ID® dye after the injection of human collagen- microparticles (**Figure 5****, panel C**). The difference with sham-treated mice was significant (87±8 vs 7±1%, p<0.0001). Autophagy induction was associated with adherence of human microparticles to mouse neutrophils (**Figure 5****, panel D**). Neutrophil vesicular fusion and degranulation depend on autophagy. Almost all circulating autophagic neutrophils expressed on their plasma membrane the MPO enzyme, which in normal conditions is retained within primary granules (**Figure 5****, panel D;** 94±3% vs 5±3% in sham-treated mice). Liposome-encapsulated clodronate reduced the interaction of neutrophils with microparticles, induction of autophagy, MPO membrane expression and the formation of NETs.

### Result 6 - Scleroderma microparticles cause endothelial damage in NSG mice

Endothelial damage is a hallmark of SSc. This example tested whether human SSc microparticles could cause endothelial damage in NSG mice.

Human collagen- microparticles were first injected into the mice. Lung inflammation developed, with massive infiltration of granulocytes expressing MPO in close contact with the vascular wall, including vessel endothelial linings, in the interstitial perivascular spaces and possibly within the alveoli (**Figure 5****, panels E and H**). Diffuse congestion and thickening of the alveolar walls was also detected by hematoxylin and eosin histochemistry (**Figure 5****, panels F and I**). Endothelial damage and lung neutrophil infiltration after injection of microparticles was associated with elevation of the collagen content in the septal and perivascular spaces, as assessed after Sirius Red staining, suggesting incipient fibrosis (**Figure 5****, panels G and J**)**.** Dramatically increased blood levels of soluble E-selectin, the specific marker of endothelial activation, whose concentration is persistently elevated in the plasma of patients with SSc, also confirmed the endothelial response to microparticles injection (**Figure 5****, panel K**). The citrullinated H4 histone concentration in cell-free plasma also was strikingly higher in microparticles-treated than in sham-treated mice (**Figure 5****, panel L**), suggesting active generation and accumulation of NETs.

SSc- microparticles injected into NSG mice also induced neutrophil autophagy and redistribution of MPO (62±9% and 78±5% of the total blood neutrophils respectively after 18 hours; **Figure 5****, panels M and N;** each symbol refers to microparticles of a single patient injected in a single mouse) (**Table 2**). In sharp contrast, the injection of microparticles retrieved from the blood of healthy donors (control-microparticles) did not influence the autophagic flux or the expression of MPO (10±2% and 11±1% respectively; statistically different from SSc-microparticles injected mice, both p<0.0001, but not from sham-treated mice, 7±1% and 5±3% respectively) (**Figure 5****, panel N.** The HMGB1 antagonist, BoxA, abrogated the induction of autophagy, the redistribution of MPO at the plasma membrane and the accumulation in the blood of NET byproducts elicited by SSc-microparticles (**Figure 5****, panel O**), indicating that microparticle-associated HMGB1 was required for the *in vivo* effects of microparticles. The plasma concentration of NET byproducts -soluble citrullinated histones and low molecular weight DNA fragments - also increased significantly after the injection of SSc- microparticles. Control-microparticles did not exert detectable effects (**Figure 5****, panel P**). Microparticles retrieved from the blood of patients with SLE were weaker inducers of neutrophil activation compared with SSc-microparticles (**Table 2**), possibly because of the substantially lower fraction expressing HMGB1 (**Figure 1** and **Table 2**).

### Summary of the Examples

The results indicate that platelet-derived microparticles expressing HMGB1 are a biomarker for the diagnosis of systemic sclerosis and an increased level of such microparticles as compared with a control indicates a diagnosis of systemic sclerosis. The results further support the other related applications (assessing disease progression, assessing efficacy of therapy etc) as claimed.

The results suggest that systemic sclerosis could be treated with an antibody against HMGB1 which would bind to the platelet-derived microparticles expressing HMGB1 in the blood of systemic sclerosis patients.

### SEQUENCE LISTING

SEQ ID No. 1
   Sequence of HMGB1 in humans:

MGKGDPKKPRGKMSSYAFFVQTCREEHKKKHPDASVNFSEFSKKCSERWKTMSAKEKGKFEDMAKADKARYERE
MKTYIPPKGETKKKFKDPNAPKRPPSAFFLFCSEYRPKIKGEHPGLSIGDVAKKLGEMWNNTAADDKQPYEKKAAK
LKEKYEKDIAAYRAKGKPDAAKKGVVKAEKSKKKKEEEEDEEDEEDEEEEEDEEDEDEEEEDDDD

### BIBLIOGRAPHY

C. P. Denton, D. Khanna, Systemic sclerosis. Lancet 390, 1685-1699 (2017)
A. Gabrielli, E. V. Avvedimento, T. Krieg, Scleroderma. N Engl J Med 360, 1989-2003 (2009)
A. J. Gilbane, C. P. Denton, A. M. Holmes, Scleroderma pathogenesis: a pivotal role for fibroblasts as effector cells. Arthritis research & therapy 15, 215 (2013)
P. C. Grayson, M. J. Kaplan, At the Bench: Neutrophil extracellular traps (NETs) highlight novel aspects of innate immune system involvement in autoimmune diseases. Journal of leukocyte biology 99, 253-264 (2016)
H. E. Harris, U. Andersson, D. S. Pisetsky, HMGB1: A multifunctional alarmin driving autoimmune and inflammatory disease. Nature reviews. Rheumatology 8, 195-202 (2012)
K. Hayakawa, L. D. Pham, Z. S. Katusic, K. Arai, E. H. Lo, Astrocytic high-mobility group box 1 promotes endothelial progenitor cell-mediated neurovascular remodeling during stroke recovery. Proc Natl Acad Sci U S A 109, 7505-10 (2012)
A. Ilich, I. Bokarev, N. S. Key, Global assays of fibrinolysis. International journal of laboratory hematology 39, e142-e143 (2017)
A. A. Manfredi, P. Rovere-Querini, A. D'Angelo, N. Maugeri, Low molecular weight heparins prevent the induction of autophagy of activated neutrophils and the formation of neutrophils extracellular traps. Pharmacol Res 123, 146-156 (2016)
N. Maugeri, P. Rovere-Querini, V. Evangelista, C. Covino, A. Capobianco, M. T. Bertilaccio, A. Piccoli, L. Totani, D. Cianflone, A. Maseri, A. A. Manfredi, Neutrophils phagocytose activated platelets in vivo: a phosphatidylserine, P-selectin, and {beta}2 integrin-dependent cell clearance program. Blood 113, 5254-5265 (2009)
N. Maugeri, S. Franchini, L. Campana, M. Baldini, G. A. Ramirez, M. G. Sabbadini, P. Rovere-Querini, A. A. Manfredi, Circulating platelets as a source of the damage-associated molecular pattern HMGB1 in patients with systemic sclerosis. Autoimmunity 45, 584-587 (2012)
A. Rouhiainen, S. Imai, H. Rauvala, J. Parkkinen, Occurrence of amphoterin (HMG1) as an endogenous protein of human platelets that is exported to the cell surface upon platelet activation. Thromb Haemost 84, 1087-1094 (2000)
R. M. Silver, J. F. Metcalf, J. H. Stanley, E. C. LeRoy, Interstitial lung disease in scleroderma. Analysis by bronchoalveolar lavage. Arthritis Rheum 27, 1254-1262 (1984)

## Claims

1. Use of platelet-derived microparticles expressing HMGB1 as a biomarker for the diagnosis of systemic sclerosis, assessment of severity and progression of systemic sclerosis and assessment of the efficacy of therapy for systemic sclerosis.

2. A method of diagnosing systemic sclerosis in an individual suspected of having systemic sclerosis, comprising:
(a) determining the level of platelet-derived microparticles expressing HMGB1 as a biomarker in a blood derived sample obtained from the individual;
(b) comparing the level of the biomarker in the blood derived sample with a control value, such that a higher level of the biomarker in the blood derived sample obtained from the individual as compared with the control value is indicative of a diagnosis of systemic sclerosis in the individual.

3. A method of determining the severity of systemic sclerosis in a patient diagnosed with systemic sclerosis, comprising:
(a) determining the level of platelet-derived microparticles expressing HMGB1 as a biomarker in a blood derived sample obtained from the patient;
(b) comparing the level of the biomarker in the blood derived sample with a control value, such that the level of the biomarker in the blood derived sample obtained from the patient is higher than that of the control value, whereby the higher the level of the biomarker in the blood derived sample obtained from the patient as compared with the control value the greater the severity of the systemic sclerosis in the patient.

4. A method of assessing the progression of systemic sclerosis in a patient suffering from systemic sclerosis, comprising:
(a) determining the level of platelet-derived microparticles expressing HMGB1 as a biomarker in a blood derived sample obtained from the patient;
(b) comparing the level of the biomarker in the blood derived sample obtained from the patient with a control value, such that the level of the biomarker in the blood derived sample obtained from the patient as compared with the control value is indicative of disease severity in the patient;
(c) comparing the level of the biomarker in the blood from the patient over time, so as to evaluate the extent or the rate of disease progression whereby an increase in the level of the biomarker in the blood derived sample obtained from the patient as compared with the control value is indicative of an increased disease severity in the patient and a reduction in the level of the biomarker in the blood derived sample obtained from the patient as compared with the control value is indicative of an reduced disease severity in the patient.

5. A method of assessing the efficacy of a therapy for systemic sclerosis in a patient suffering from systemic sclerosis, comprising:
(a) determining the level of platelet-derived microparticles expressing HMGB1 as a biomarker in a blood derived sample obtained from the patient;
(b) comparing the level of the biomarker in the blood derived sample with a control value, such that the level of the biomarker in the blood derived sample obtained from the individual as compared with the control value is indicative of disease severity in the patient;
(c) treating the patient with the therapy for systemic sclerosis;
(d) comparing the level of the biomarker in the blood from the patient after treatment, so as to evaluate the extent or the rate of disease progression whereby a reduction in the level of the biomarker in the blood derived sample obtained from the individual as compared with the control value is indicative of the therapy being efficacious in the treatment of systemic sclerosis in the patient.

6. A method of assessing the efficacy of a therapy in a patient having or suspected of having systemic sclerosis, comprising measuring at least two different time points, in a sample from said patient, the level of platelet-derived microparticles expressing HMGB1 as a biomarker whereby a reduction in the level of the biomarker with time indicates that the therapy is efficacious.

7. The method according to any one of claims 2 to 6 wherein determining the level of the biomarker is performed by a process comprising use of anti-HMGB1 antibodies to bind to HMGB1 expressed by the microparticles.

8. The method according to claim 7 wherein the determining the level of the biomarker further comprises the use of a secondary antibody against the anti-HMGB1 antibodies.

9. The method according to claim 7 wherein determining the level of the biomarker is performed by a process comprising using anti-HMGB1 antibodies to bind to HMGB1 expressed by the microparticles and using another type of antibody which bind to another marker expressed by platelet derived microparticles.

10. Use of a kit comprising a biosensor capable of detecting and/or determining the level of a platelet-derived microparticle expressing HMGB1 as a biomarker for monitoring or diagnosing systemic sclerosis in an individual.

11. A method of treatment of systemic sclerosis in a patient suffering from systemic sclerosis, which comprises the step of administering an effective amount of anti-HMGB1 antibody to said patient.

12. An anti-HMGB1 antibody for use in the treatment of systemic sclerosis in a patient.

13. The method or antibody for use according to claim 11 or 12 wherein the patient has been identified as having an increased level of platelet-derived microparticles expressing HMGB1 as a biomarker in a blood-derived sample from said patient when compared to a control value.

14. A method of treating a patient suffering from systemic sclerosis, which comprises the following steps:
(a) determining the level of platelet-derived microparticles expressing HMGB1 as a biomarker in a blood derived sample obtained from the patient;
(b) comparing the level of the biomarker in the blood derived sample with a control value, whereby a higher level of the biomarker in the blood derived sample obtained from the patient as compared with the control value indicates a diagnosis of systemic sclerosis in the patient; and
(c) administering an anti-HMGB1 antibody to a patient diagnosed in step (b) as a patient with systemic sclerosis.

15. An anti-HMGB1 antibody for use in the treatment of systemic sclerosis in a patient who has been diagnosed with systemic sclerosis and has an increased level of platelet-derived microparticles expressing HMGB1 as a biomarker in a blood derived sample obtained from the patient as compared with the level of the biomarker in a blood derived sample obtained from a normal individual.


SEQUENCE LISTING

<110>  Bianchi, Marco

<120>  NOVEL BIOMARKERS

<130>  MAB-P2412EPp

<160>  1

<170>  PatentIn version 3.5

<210>  1
<211>  214
<212>  PRT
<213>  Homo sapiens

<400>  1

Met Gly Lys Gly Asp Pro Lys Lys Pro Arg Gly Lys Met Ser Ser Tyr
1               5                   10                  15

Ala Phe Phe Val Gln Thr Cys Arg Glu Glu His Lys Lys Lys His Pro
            20                  25                  30

Asp Ala Ser Val Asn Phe Ser Glu Phe Ser Lys Lys Cys Ser Glu Arg
        35                  40                  45

Trp Lys Thr Met Ser Ala Lys Glu Lys Gly Lys Phe Glu Asp Met Ala
    50                  55                  60

Lys Ala Asp Lys Ala Arg Tyr Glu Arg Glu Met Lys Thr Tyr Ile Pro
65                  70                  75                  80

Pro Lys Gly Glu Thr Lys Lys Lys Phe Lys Asp Pro Asn Ala Pro Lys
                85                  90                  95

Arg Pro Pro Ser Ala Phe Phe Leu Phe Cys Ser Glu Tyr Arg Pro Lys
            100                 105                 110

Ile Lys Gly Glu His Pro Gly Leu Ser Ile Gly Asp Val Ala Lys Lys
        115                 120                 125

Leu Gly Glu Met Trp Asn Asn Thr Ala Ala Asp Asp Lys Gln Pro Tyr
    130                 135                 140

Glu Lys Lys Ala Ala Lys Leu Lys Glu Lys Tyr Glu Lys Asp Ile Ala
145                 150                 155                 160

Ala Tyr Arg Ala Lys Gly Lys Pro Asp Ala Ala Lys Lys Gly Val Val
                165                 170                 175

Lys Ala Glu Lys Ser Lys Lys Lys Lys Glu Glu Glu Glu Asp Glu Glu
            180                 185                 190

Asp Glu Glu Asp Glu Glu Glu Glu Glu Asp Glu Glu Asp Glu Asp Glu
        195                 200                 205

Glu Glu Asp Asp Asp Asp
    210